# EUROPÄISCHE PATENTSCHRIFT

(11) **EP 2 221 087 B1**
(45) Veröffentlichungstag und Bekanntmachung des Hinweises auf die Patenterteilung: **07.09.2016**
(21) Anmeldenummer: 10152262.1
(22) Anmeldetag: 01.02.2010
(51) Int. Cl.: A61N 1/378

(54) **Aktives medizinisches Implantat**
Active medical implant
Implant médical actif

(30) Priorität: 20.02.2009 DE 102009001042
(43) Veröffentlichungstag der Anmeldung: 25.08.2010
(73) Patentinhaber: Biotronik CRM Patent AG, 6341 Baar (CH)
(72) Erfinder: Weiss, Ingo, 12435, Berlin (DE); Knorr, Stefan, 10119, Berlin (DE)
(74) Vertreter: Galander, Marcus

(56) Entgegenhaltungen:
- WO-A2-2007/109272
- DE-A1-102006 062 541
- US-A- 3 943 936
- US-A- 5 628 777
- US-A1- 2005 256 549
- US-A1- 2006 136 005
- US-A1- 2006 217 776

## Beschreibung

Die Erfindung betrifft ein aktives medizinisches Implantat, insbesondere ein medizinelektronisches Gerät, wie etwa einen Herzschrittmacher, einen implantierbaren Defibrillator, eine implantierbare Medikamentendosierpumpe oder ein ähnliches Gerät.

Aktive medizinische Implantate besitzen zur Energieversorgung entweder einen Energiespeicher (Batterie, Akku, Kondensator) oder werden permanent transkutan mit Energie versorgt (über Kabel, HF, wechselndes Magnetfeld). Transkutane Energieversorgung ist nur kurzfristig einsetzbar, da ein hohes Infektionsrisiko besteht oder da die permanente HF-Belastung zu Gewebeschädigungen führen kann. Sie hat ich daher in der breiten medizinischen Praxis nicht durchsetzen können.

Energiespeicher sind nach einer gewissen Zeit erschöpft und müssen ersetzt oder geladen werden. Die Ersetzung des Energiespeichers ist notwendigerweise mit einer Explantation des gesamten Gerätes und Neuimplantation desselben bzw. eines Ersatzgerätes verbunden. Diese Vorgehensweise war in der Vergangenheit die weitest Verbreitete, ist jedoch aufwändig, teuer und auch mit medizinischen Risiken und erheblichen Unbequemlichkeiten für den Träger des Implantates verbunden.

Zum Laden eines aufladbaren Energiespeichers (Akkus) eines aktiven Implantats wird üblicherweise eine extracorporale Primärspule und eine im Implantat befindliche Sekundärspule genutzt. Die Energie wird durch ein magnetisches Wechselfeld von der Primäran die Sekundärspule übertragen. Die maximal übertragbare Energie wird durch die Erwärmung des Gewebes beim Energiedurchgang begrenzt. Ein Ladezyklus des Energiespeichers kann durch eine geringe Energieübertragung sehr lang werden. Gängige wiederaufladbare chemische Zellen besitzen eine längere Lebensdauer, wenn sie regelmäßig und häufig geladen werden und so stets einen hohen Ladezustand besitzen. Je nach Anwendung der transcuten Energieübertragung kann die wiederaufladbare Zelle vor dem Ladevorgang sehr stark entladen werden, was die Lebensdauer der Zelle reduziert. Die Einhaltung von regelmäßigen Ladezyklen zum Funktionserhalt des Implantates liegt in der Verantwortung des Arztes oder des Patienten. Sie erfordert in der Praxis des Arztes erheblichen organisatorischen Aufwand und vom Patienten Disziplin und ist in der Praxis oft nicht hinreichend gewährleisten.

Aus US 3,946,936 ist ein Implantat gemäß erstem Teil des Anspruchs 1 bekannt, mit einem schwingungsfähigen mechanischen System offenbart, welches durch Bewegungen des Implantat-Trägers in Schwingung versetzt werden kann und dadurch die zum Betrieb des Implantats notwendige Energie erzeugt.

Der Erfindung liegt daher die Aufgabe zugrunde, ein hinsichtlich seiner Energieversorgung verbessertes medizinisches Implantat bereitzustellen, dessen Betriebsfähigkeit insbesondere mit geringem medizinischem, technischem und organisatorischem Aufwand dauerhaft gesichert ist.

Diese Aufgabe wird durch ein aktives medizinisches Implantat mit den Merkmalen des Anspruchs 1 gelöst. Zweckmäßige Fortbildungen des Erfindungsgedankens sind Gegenstand der abhängigen Ansprüche.

Erfindungsgemäß wird in dem Implantat ein Schwingungsfähiges mechanisches System eingesetzt. Die mechanischen Schwingungen werden insbesondere durch einen mechanischelektrischen Wandler in elektrische Energie umgewandelt, und diese steht dem Implantat zur Verfügung. Als mechanisch-elektrische Wandler können z.B. piezoelektrische Materialien oder elektromagnetische Wandler eingesetzt werden.

Diese elektrische Energie kann zum Laden eines Energiespeichers, etwa eines Akkus oder Energiespeicher-Kondensators, oder zur teilweisen Deckung des permanenten Energieverbrauchs des Implantates genutzt werden. Alternativ kann auch vorgesehen sein, dass der Energiespeicher einen Speicher mechanischer Energie, insbesondere ein Schwungrad oder dergleichen, aufweist und diesem ein mechanisch-elektrischer Wandler zur Umwandlung gespeicherter mechanischer Energie in elektrische Betriebsenergie des Verbrauchers zugeordnet ist.

Es kann vorgesehen sein, dass der Rotor Schwinges mit elektrischen Wirkkomponenten des Implantats in einem gemeinsamen Gehäuse angeordnet ist. Das schwingungsfähige System kann aber auch außerhalb des aktiven Implantates als eigenes IMD implantiert werden und die Energie an die Elektronik des aktiven Implantates übertragen. So kann das schwingungsfähige System z.B. näher an einem Knochen befestigt werden und besser an energetisch ergiebige Bewegungen des Körpers koppeln.

In einer weiteren Ausführung sind mehrere Schwinger mit unterschiedlichen Bewegungsachsen und/oder unterschiedlicher Eigenfrequenz vorgesehen. Alternativ oder in Kombination hierzu kann vorgesehen sein, dass der oder ein Schwinger in mehr als einer Richtung, insbesondere dreidimensional, schwingungsfähig ausgebildet ist.

Schließlich ist, gleichfalls zur optimalen Ausnutzung von verschiedenen Bewegungen des Patienten in unterschiedlichen Körperlagen zur Energiegewinnung für das Implantat, eine Ausführung möglich, bei der der oder ein Schwinger eine selbsttätig veränderliche Drehachse aufweist. Insbesondere ist hierbei vorgesehen, dass der Schwinger mit veränderlicher Drehachse Mittel zur Einstellung der Drehachse in Abhängigkeit von der Körperlage und/oder von Bewegungen des Implantat-Trägers aufweist.

Im Hinblick auf die konstruktive Realisierung der mechanischen Energiequelle ist in einer weiteren Ausführung der Erfindung vorgesehen, dass der oder ein Schwinger Funktions- und/oder Tragkomponenten des Implantats umfasst, die drehbar in einem Gehäuse aufgehängt sind. Speziell kann das so aussehen, dass der oder ein Schwinger einen Tragrahmen des Implantats mit darin gelagerten Funktionskomponenten umfasst.

So kann die Masse des Schwingers erhöht und die Eigenfrequenz verringert werden. Das kann soweit gehen, dass das schwingungsfähige System durch den gesamten Innenaufbau des Implantates (Energiespeicher, elektrische Schaltung, Montierrahmen, ...) gebildet wird.

Eine weitere Ausführung, die auf eine möglichst effiziente Ausnutzung der Energie verschiedener Bewegungen des Patienten zur Versorgung des Implantats abzielt, sieht vor, dass der oder jeder Schwinger zur Aufnahme einer äußeren Anregung mit einer auf seine Eigenfrequenz abgestimmten Frequenz ausgebildet ist. Hierbei ist speziell dem äußerlich anregbaren Schwinger ein elektrisch-mechanischer Wandler, insbesondere ein Piezoelement oder elektromagnetischer Wandler, zur Anregung durch ein äußeres elektromagnetisches Wechselfeld zugeordnet.

Die Erfindung ermöglicht unter anderem:
- den Bau von aktiven Implantaten mit einer Primärbatterie, die eine höhere Lebensdauer als bisher besitzen,
- den Bau von aktiven Implantaten mit einer wiederaufladbaren Zelle, wobei die Energie zum Laden des Akkus aus Bewegungen des Patienten gewonnen wird,
- den Bau von Implantaten mit höherer Lebenserwartung als bisher, ohne dass das Implantat auf regelmäßige Ladevorgänge von außen angewiesen ist. Dadurch entfällt auch die thermische Belastung des Gewebes beim Laden (z.B. durch Wirbelströme im metallischen Gehäuse, die durch ein magnetisches Wechselfeld induziert werden).

Hinsichtlich der Dimensionierung des mechanischen Systems ist es von Vorteil, wenn die Masse des schwingungsfähigen Systems mehr als 5% der Gesamtmasse des Implantates ausmacht. Des Weiteren kann es von Vorteil sein, wenn das System eine Eigenfrequenz von weniger als 10 Hz aufweist.

Vorteile und Zweckmäßigkeiten der Erfindung ergeben sich im Übrigen aus der nachfolgenden Beschreibung bevorzugter Ausführungsbeispiele anhand der Figuren. Von diesen zeigen:
- Fig. 1A und 1B: eine schematische Darstellung einer ersten, nicht zum Gegenstand der Patentansprüche gehörenden Ausführung des erfindungsgemäßen Implantats,
- Fig. 2: eine schematische Darstellung einer zweiten, nicht zum Gegenstand der Patentansprüche gehörenden Ausführung des erfindungsgemäßen Implantats,
- Fig. 3: eine detailliertere Darstellung einer ersten Ausgestaltung der nicht zum Gegenstand der Patentansprüche gehörenden ersten Ausführung,
- Fig. 4: eine detailliertere Darstellung einer zweiten Ausgestaltung der nicht zum Gegenstand der Patentansprüche gehörenden ersten Ausführung,
- Fig. 5: eine detailliertere Darstellung einer dritten Ausgestaltung der nicht zum Gegenstand der Patentansprüche gehörenden ersten Ausführung,
- Fig. 6: eine detailliertere Darstellung einer vierten Ausgestaltung der nicht zum Gegenstand der Patentansprüche gehörenden ersten Ausführung,
- Fig. 7: eine Darstellung zur Platzierung und Funktion der letztgenannten nicht zum Gegenstand der Patentansprüche gehörenden Ausführung im Körper eines Patienten,
- Fig. 8: eine schematische Darstellung einer gegenüber der letztgenannten Konstruktion modifizierten Ausgestaltung der ersten nicht zum Gegenstand der Patentansprüche gehörenden Ausführungsform,
- Fig. 9: eine weitere modifizierte, nicht zum Gegenstand der Patentansprüche gehörenden Ausgestaltung dieses Implantats,
- Fig. 10: eine Darstellung einer weiteren, nicht zum Gegenstand der Patentansprüche gehörenden modifizierten Ausgestaltung und
- Fig. 11 bis 13: Darstellungen verschiedener Ausgestaltungen einer weiteren Ausführung der Erfindung.

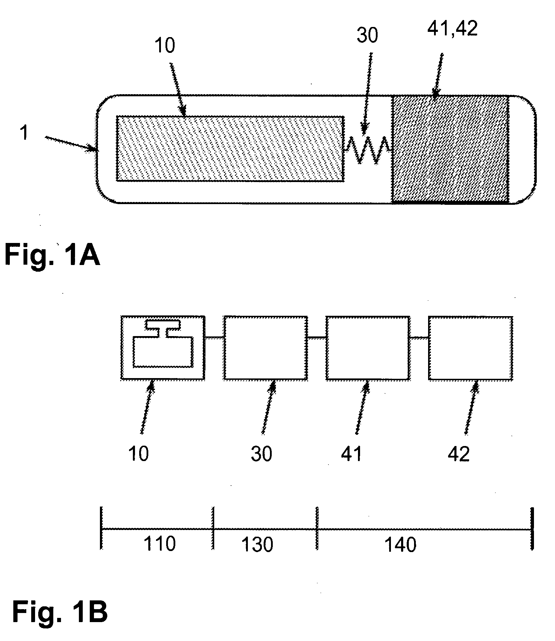

Fig. 1A zeigt ein aktives Implantat 1 mit einem Gehäuse 1a, das eine bewegliche Teilmasse 10 enthält. Die Masse 10 ist über ein Kopplungssystem 30 mit weiteren fest im Gehäuse installierten Komponenten 41, 42 oder dem Gehäuse 1a selbst verbunden. Das Kopplungssystem wandelt die Energie der mechanischen Schwingung in elektrische Energie und stellt diese Leistung der Implantatelektronik 41 zur Verfügung. Die Energie kann in einem Energiereservoir 42 gespeichert werden. Dieses Reservoir kann eine elektrochemische Zelle oder ein Kondensator sein.

Physikalisch kann das System, wie in Fig. 1B dargestellt, beschrieben werden durch eine schwingungsfähige Masse 10, die mit einem mechanoelektrischen Wandler 30, einer elektrischen Schaltung 41 und einem Energiereservoir 42 gekoppelt ist. Im ersten Abschnitt des Systems kann die Masse Bewegungen ausführen. Die Energie der Bewegungen wird im zweiten Abschnitt 130 durch den mechanoelektrischen Wandler in elektrische Energie umgewandelt. Im letzten Abschnitt 140 wird die elektrische Energie von der Implantatelektronik 41 genutzt und/oder im Energiereservoir 42 gespeichert.

Das Kopplungssystem enthält neben dem mechanoelektrischen Wandler noch ein (hier nicht dargestelltes) Rückstellsystem, das die bewegliche Masse in eine bestimmte Ruheposition bringt. Ein solches Rückstellsystem kann eine Feder beliebiger Bauform beinhalten. Besonders geeignet sind Blattfedern, da sie eine geringe Bauhöhe besitzen.

Zur Rückstellung können auch andere Prinzipien genutzt werden, z.B. zwei Magnete, die sich abstoßen. Ein solches System ist im Gegensatz zu einer Feder nichtlinear und kann somit ein breitbandiges Schwingungsverhalten hervorrufen. Die Federn können bevorzugt aus nichtmagnetischem Material bestehen, etwa aus Messinglegierungen, Titanlegierungen, Kunststoff oder Keramik. Grundsätzlich kann die Schwingung um jede beliebige Achse im Implantat oder entlang jeder beliebigen Achse erfolgen.

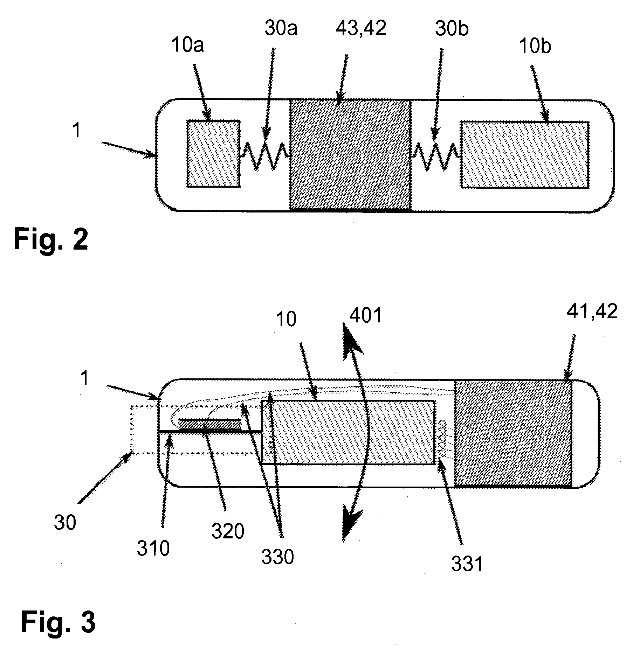

Ein in Fig. 2 schematisch dargestelltes Implantat mit mehreren schwingungsfähigen Teilsystemen arbeitet grundsätzlich wie das oben vorgestellte System. Es gibt allerdings zwei Massen 10a, 10b und zwei zugehörige Kopplungssysteme 30a, 30b. Die Implantatelektronik 43 ist dazu ausgebildet die elektrische Energie von mehr als nur einem Kopplungssystem zu nutzen.

Etwas genauer wird die Ausführung eines Implantats mit einem schwingungsfähigen Teilsystem in Fig. 3 gezeigt und nachfolgend beschrieben. Die schwingungsfähige Masse 10 ist über ein Kopplungssystem 30 mit dem Implantatgehäuse verbunden. Das Kopplungssystem besteht aus einer Blattfeder 310 und einem piezoelektrischen Element 320. Dieses Wandlerelement wandelt die mechanische Energie in elektrische Energie. Die beiden Anschlüsse des Elements 320 sind über elektrische Leiter 330 mit der Implantatelektronik 41 verbunden. Die elektrischen Verbindungen 331 werden vorzugsweise dort angebracht, wo die mechanische Beanspruchung durch das schwingende Teilsystem am geringsten ist. Wenn die Masse 10 teilweise aus elektrischen Komponenten der Implantatelektronik 41 oder das Energiereservoirs 42 besteht, werden mehrere bewegliche, elektrische Verbindungen 331 zwischen der schwingungsfähigen Masse 10 und den fest installierten Teilen der Elektronik genutzt. Die Figuren sind insofern nur als Prinzipskizzen zu verstehen.

Die Masse 110 kann sich wie dargestellt 401 bewegen. Durch die Bewegung der Masse 10 verformt sich die Feder 310 und damit der darauf montierte piezoelektrische Wandler 320. Im Innern des Wandlers baut sich ein starkes elektrisches Feld auf und zwischen den beiden Kontakten baut sich eine Spannung auf. Schwingt die Masse 10 hin und her, so liegt an den Kontakten des Wandlers 320 eine Wechselspannung an.

Statt eines piezoelektrischen Elements 320 kann auch ein Stapel mehrerer piezoelektrischer Elemente verwendet werden. Statt einzelner Keramikscheiben können auch piezoelektrische Fasern genutzt werden, z.B. als sog. "macro fiber composite". Je nach Ausführung des piezoelektrischen Wandlers 320 kann er auch die Funktion der Feder 310 übernehmen und diese ersetzen.

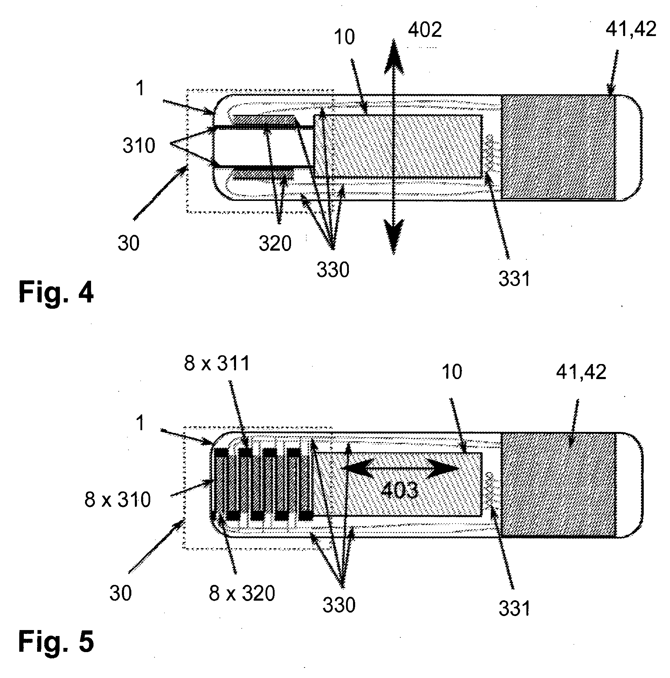

Statt einer Feder können, wie in Fig. 4 gezeigt, auch mehrere Federn 310 genutzt werden. Im Beispiel wird so die Bewegung der Masse 10 von einer Rotation 401 in eine Translation 402 verändert. Die piezoelektrischen Wandler 320 müssen nicht auf allen Federn 310 montiert sein.

Die Kombination mehrerer Blattfedern in Form eines Stapels ist, wie in Fig. 5 gezeigt, ebenfalls möglich. Im Beispiel sind acht Federn 310 mit je einem piezoelektrischen Wandler 320 kombiniert und ermöglichen eine Translation 403 der Masse 10. Je vier Piezoelemente sind elektrisch parallel geschaltet. Eine Reihenschaltung ist ebenfalls möglich. Die steifen Verbindungen 311 der Federn verformen sich bei der Bewegung 403 nur minimal, damit die Hauptverformung in den Federn 310 und damit auch in den piezoelektrischen Elementen 320 erfolgt.

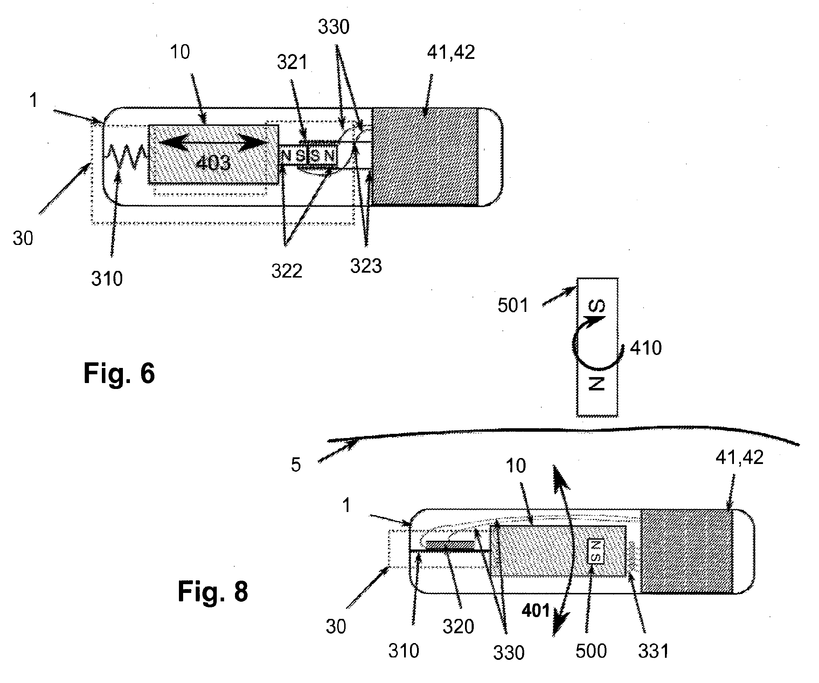

Ein Implantat mit einem mechanoelektrischen Wandler kann auch den in Fig. 6 dargestellten und nachfolgend beschriebenen Aufbau besitzen. Die Masse 10 ist über das Kopplungssystem 30 mit dem Implantatgehäuse 1 verbunden. Das Kopplungssystem besteht aus zwei Komponenten: dem Federelement 310 und einem elektromagnetischen Wandler, der durch eine Spule 321, zwei Magnete 322, eine Halterung 323 und elektrische Leitungen 330 gebildet wird. Durch die Bewegung 403 der Masse 10 bewegen sich die Magnete 322, und in der Spule 321 wird eine Spannung induziert. Diese wird über die Leitungen 330 zur Implantatelektronik 41 übertragen und kann dort genutzt oder im Energiereservoir 42 gespeichert werden.

Auch dieser Aufbau ist sehr variabel, und die Anzahl und Anordnung der Magnete und Spulen kann vielfältig geändert werden. So können z.B. die Spulen an der Masse befestigt und die Magnete fest mit dem Implantat verbunden sein. Hierdurch werden Wirbelstromverluste durch die Bewegung der Magnete vermieden. Das Federelement 310 und der mechanoelektrische Wandler können z.B. auch koaxial angeordnet sein.

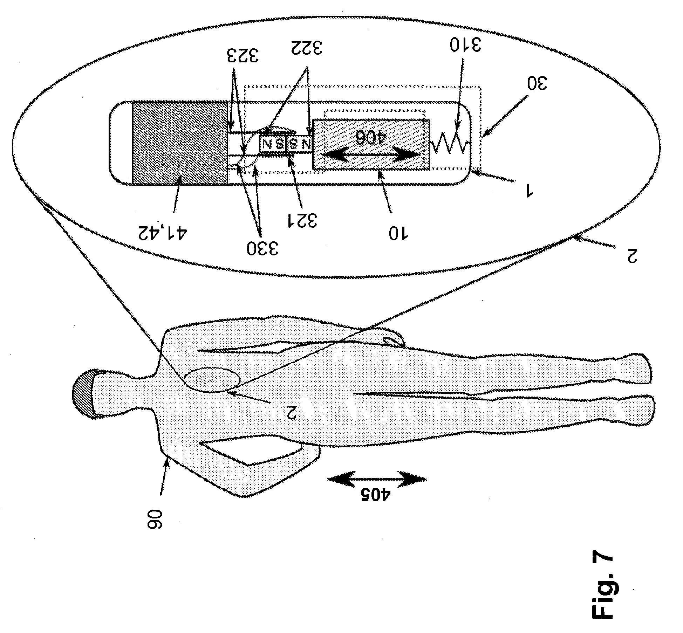

Die Funktionsweise eines Implantates mit einer schwingungsfähigen Masse im Patienten ist, wie in Fig. 7 skizzenartig dargestellt, folgende: Ein Patient 90 mit einem aktiven Implantat 1 in einem Körperbereich 2 bewegt sich auf- und abwärts 405, z.B. durch Laufen. Da das Gehäuse 1 des Implantates unmittelbaren Kontakt zum Körper des Patienten 90 besitzt, wird es ebenfalls bewegt. Die bewegliche Masse 10 im Implantat bewegt sich dann relativ 406 zum Implantatgehäuse und der mechanoelektrische Wandler erzeugt elektrische Energie.

Ist der Patient inaktiv und die Eigenbewegung schwach, so kann z.B. durch eine speziell zu dieser zweckerzeugten Bewegung des Bettes eine Relativbewegung 406 der Masse 10 gegenüber dem Implantatgehäuse hervorgerufen und dadurch im Implantat elektrische Energie erzeugt werden.

Die bewegliche Masse 10 kann auch, wie in Fig. 8 skizziert, durch andere Wechselfelder zum Schwingen angeregt werden. Beispielsweise kann ein Magnet 500 Teil der beweglichen Masse sein. Durch die Änderung des Magnetfelds kann eine Kraft oder ein Moment auf die Masse 10 übertragen werden, diese bewegt sich und der Wandler erzeugt elektrische Energie. Die Änderung des Magnetfeldes kann durch die Bewegung eines starken Magneten außerhalb des Patienten erzeugt werden. Die Abbildung zeigt einen Magneten 501, der sich außerhalb des Körpers 5 befindet und in der durch den Pfeil 410 bezeichneten Weise gedreht wird.

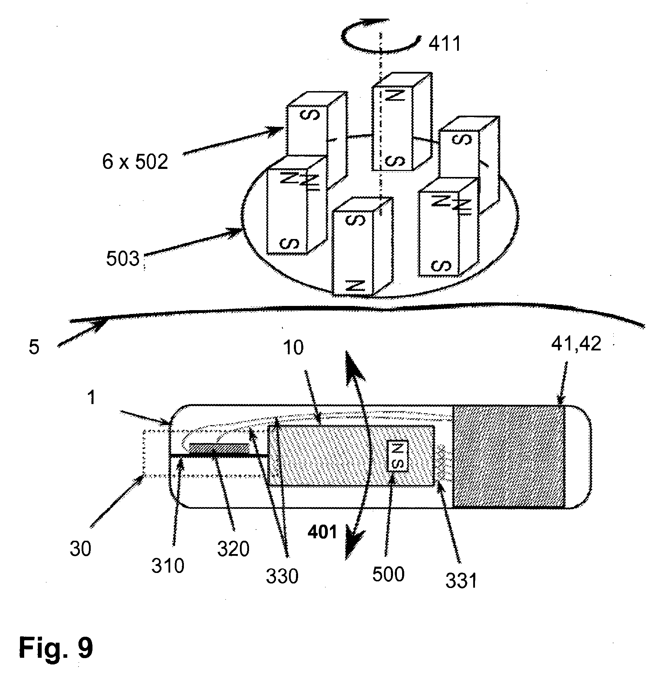

Alternativ können auch mehrere Magnete mit alternierenden Ausrichtungen über dem Implantat vorbeibewegt werden, wie in Fig. 9 dargestellt. Außerhalb des Körpers 5 befinden sich hier 6 Magnete 502, die auf einer Scheibe 503 montiert sind. Die Scheibe dreht sich in Richtung des Pfeils 411. Durch die Drehbewegung wird ein magnetisches Wechselfeld erzeugt, und die bewegliche Masse 10 kann zum Schwingen angeregt werden.

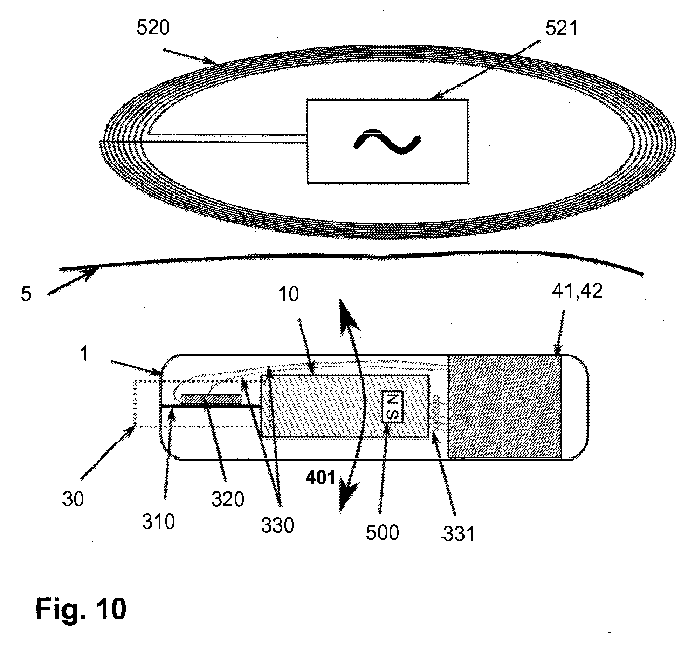

Das magnetische Wechselfeld kann auch durch einen zeitabhängigen Stromfluss durch eine Spule erzeugt werden, wie in Fig. 10 schematisch dargestellt. Ein Spannungsgenerator 521 erzeugt eine Signalform. Der Ausgang des Generators ist mit einer Spule 520 verbunden und erzeugt in der Spule einen zeitabhängigen Stromfluss, der ein zeitabhängiges Magnetfeld erzeugt. Das wechselnde Magnetfeld wechselwirkt mit dem Magneten 500 der beweglichen Masse 10 im Implantat 1 und regt die Masse zum Schwingen an. Die Schwingungsenergie wird von einem Kopplungssystem 30 in elektrische Energie umgewandelt.

Vorteil der Erzeugung von Wechselfeldern durch sich bewegende Magnete sind die niedrigen Frequenzen des Systems, die zu einer geringeren Erwärmung des Gehäuses und des Gewebes durch Wirbelströme führt.

Im Falle der Anregung des mechanischen schwingungsfähigen Systems von außen, gibt es außerhalb des Körpers die Möglichkeit zur rückkoppelnden Steuerung der mechanischen Schwingung im Inneren des Implantates. Durch solch eine Rückkopplung (closed loop) kann z.B. die Position oder die Frequenz der Erregung auf das Implantat abgestimmt werden, um eine möglichst gute Energieübertragung zu gewährleisten.

Die Übertragung (uni- oder bidirektional) der Information aus dem Implantat heraus kann z.B. induktiv oder mittels RF-Kommunikation erfolgen. Dabei kann z.B. die vom Implantat aufgenommene Leistung, Spannung oder ein ähnlicher Parameter übertragen werden.

Sinnvoll ist auch die Übertragung der Phase der entnommenen Energie, denn so kann die Erregungsfrequenz auf die Eigenfrequenz des schwingungsfähigen Systems eingestellt werden, ohne dass das Amplitudenverhältnis bestimmt werden muss. Dies funktioniert auch bei schwingungsfähigen Systemen mit geringer Güte.

Die Rückkopplung kann direkt vom Implantat zum schwingungserregenden System erfolgen, das dann z.B. die Erregungsfrequenz anpasst oder die Position optimiert. Die Rückkopplung kann aber auch durch den Patienten erfolgen. So kann z.B. das schwingungserregende System die Daten aus der Kommunikation mit dem Implantat auswerten und dem Patienten Informationen übermitteln. Dies kann z.B. durch optische oder akustische Signale erfolgen, die dem Patienten eine bessere Ladeposition aufzeigen. Als akustische Signale sind verschiedene Töne denkbar, deren Änderung (Höhe, Frequenz, Tonabfolge, Melodie...) die Qualität der Übertragung anzeigen. Es können auch Sprachinformationen ausgegeben werden.

Ein schwingungsfähiges System im Sinne der Erfindung kann durch eine Drehfeder (z.B. Spiralfeder) und eine Masse gebildet sein. Die Masse ist insbesondere so an der Spiralfeder angebracht, dass der Schwerpunkt nicht auf der Rotationsachse liegt. So kann die Drehschwingung auch durch eine lineare Bewegung angeregt werden. Der Abstand zwischen der Rotationsachse und dem Schwerpunkt der Masse ist möglichst groß, um eine niedrige Eigenfrequenz zu erhalten.

Zusätzlich zur Spiralfeder ist in einer Ausgestaltung ein piezoelektrischer Wandler in dem schwingungsfähigen System integriert. Dieser verformt sich, sobald das System schwingt und erzeugt dann elektrische Energie. Es kann auch die Spiralfeder selbst einen oder mehrere piezoelektrische Wandler enthalten, die elektrische Energie liefern.

Als mechanoelektrischer Wandler im Implantat kann auch ein elektromagnetisches System eingesetzt werden, das wie ein Dynamo funktioniert. Dabei erzeugt die Relativbewegung zwischen einem Spulensystem und einem oder mehreren Magneten ein veränderliches Magnetfeld in dem Spulensystem. Die entstehende Spannung kann zur Stromerzeugung des Implantats genutzt werden.

Der elektromagnetische Drehwandler kann so ausgeführt sein, dass das Spulensystem fest mit dem Implantat verbunden ist und das Magnetsystem drehbar gelagert ist. Das Magnetsystem kann dann von außen zu Schwingungen angeregt werden und induziert im Spulensystem eine Spannung.

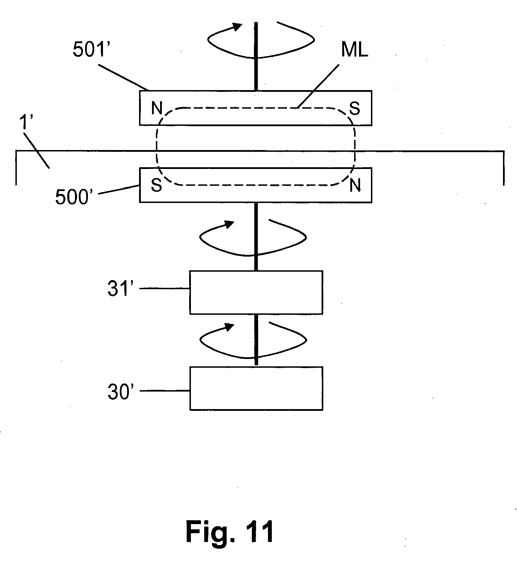

In einer weiteren Ausführung wird das Prinzip der Magnetkupplung genutzt. Ein äußerer (außerhalb des Patienten) drehender Magnet 501' nimmt, wie in Fig. 11 skizziert, einen im Implantat 1' drehbaren Magneten 500' mit. Dies funktioniert auch, wenn im Implantat 1' anstelle des Magneten nur ein magnetisches Material hoher Permeabilität verwendet wird, damit hier die Magnetfeldlinien ML gut gebündelt werden und somit die Kraft/Momentenübertragung optimal ist. Dieses magnetische Material realisiert damit eine Magnetfeldlinienbrücke (im weiteren Brücke genannt). Ein solches Metall ist in einer bevorzugten Ausführung Mu-Metall. Die Anordnung kann auch umgekehrt aufgebaut werden, nämlich so, dass sich der Magnet im Implantat befindet und außerhalb das magnetische Material hoher Permeabilität. Die bevorzugte Ausführung ist jedoch die, in der im Implantat zu diesem Zweck kein Magnet verwendet werden muss.

Damit ein hohes Drehmoment übertragen werden kann, wird vorzugsweise der Radius der im Implantat drehenden Anordnung maximal groß gewählt (d.h. so groß wie die inneren Abmessungen des Implantats dies maximal erlauben).

Um Wirbelströme zu vermeiden, muss die Drehgeschwindigkeit der Magnete gering sein. Soll diese Drehbewegung im Inneren des Implantates nun über einen elektromagnetischen Wandler (z.B. Dynamo) 30' in elektrische Spannung gewandelt werden, ist jedoch eine hohe Drehgeschwindigkeit vorteilhaft, da gemäß des Induktionsgesetzes die induzierte Spannung mit der Winkelgeschwindigkeit steigt. Dazu kann ein Aufwärtsgetriebe 31' eingesetzt werden.

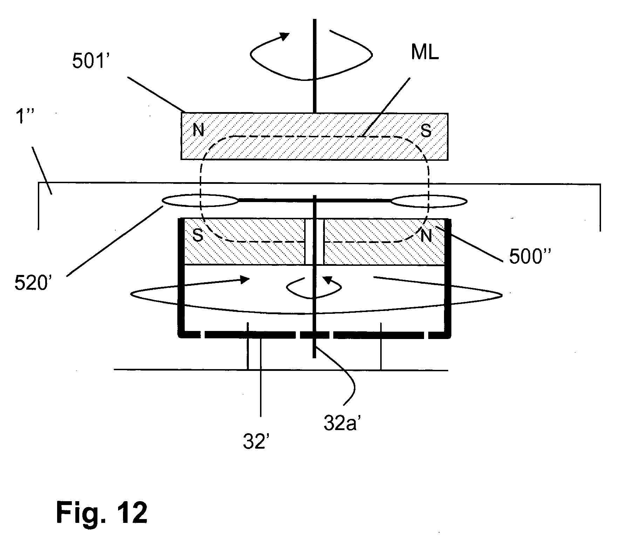

In einer weiteren Ausführung, die in Fig. 12 skizziert ist, wird das Magnetfeld, das die Drehmomentübertragung realisiert, selbst genutzt, um in Spulen 520' Spannungen zu induzieren. Diese Spulen müssen sich dann im Implantat 1" relativ zum Magnetfeld bewegen. Speziell wird dafür ein Planetengetriebe 32' eingesetzt, das zum einen als Aufwärtsgetriebe dient und zum anderen die Drehbewegung entgegen der Drehbewegung der hinteren Magnete 500" umsetzt. Somit addieren sich die Winkelgeschwindigkeiten, d.h. die Relativbewegung ist ausgeprägter, was eine höhere induzierte Spannung zur Folge hat.

Die Spannung der drehenden Spulen 520' wird über (nicht gezeigte) Schleifkontakte abgegriffen. Bevorzugt kann die Achse so realisiert werden, dass diese aus zwei elektrisch nicht verbundenen Hälften besteht, so dass die Spannung einfach über die Lager des Getriebes abgenommen werden kann. Die Lager werden bevorzugt aus Messing realisiert. Dieses bietet sowohl gute Gleiteigenschaften als auch elektrischen Kontakt. Denkbar sind auch elektrische Kontakte über ein oder mehrere Kugellager aus leitfähigen Materialien oder über leitfähige Flüssigkeiten, die einen Spalt überbrücken.

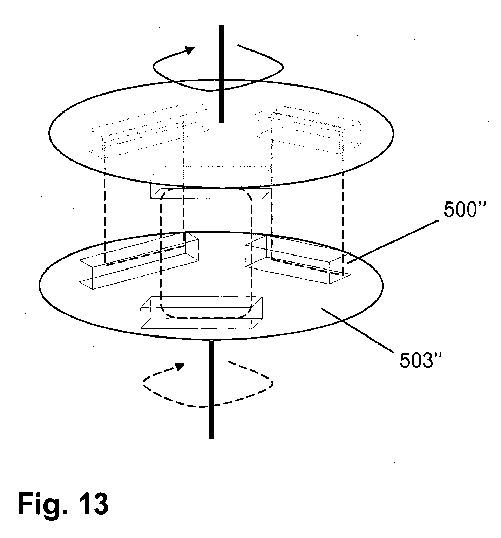

In einer weiteren vorteilhaften Anordnung werden mehrere Magnetpaare 500" oder Magnet-Brücken Paare auf jeweils einer Scheibe 503" angeordnet, wie in Fig. 13 dargestellt. Dies erhöht die Größe des übertragbaren Drehmoments.

In einer weiteren Ausführung ist ferner ein Rückkopplungsmechanismus vorgesehen, der den Schlupf regelt. Dazu wird der in den Spulen induzierte Spannungsverlauf aus dem Implantat telemetrisch nach außen kommuniziert. Aus Amplituden und Phasenlagen kann somit der Schlupf errechnet werden und die Drehgeschwindigkeit der extrakorporalen Drehung nachgeregelt werden.

Um die Bauhöhe der Spulen gering zu halten, sind plane Spulen oder Spulenanordnungen zu bevorzugen. Diese können z.B. gewickelt oder auch monolithisch hergestellt werden.

Statt der rotierenden Spulen können (in einer nicht dargestellten Ausführung) auch ortsfeste Spulen verwendet werden. Der magnetische Fluss durch die Spulenfläche wird durch die Bewegung der Magneten zeitlich verändert. Für schnelle Magnetflussänderungen kann ebenfalls das oben beschriebene Prinzip des Aufwärtsgetriebes verwendet werden. Schnell dreht sich dabei eine Magnetbrücke. Die magnetisch gekuppelten Magnete im Implantat drehen sich nur mit der Winkelgeschwindigkeit der äußeren Magnete und erzeugen keine Wirbelströme. Immer wenn die Brücke einen Magnetkreis schließt, ändert sich in der Spule, die sich gerade im Spalt befindet, der Magnetfluss sehr stark, eine hohe Spannung wird induziert. Vorteil dieser Ausführung ist neben der elektrischen Kontaktierung der Spulen die Positionierung der Spulen. Diese können in einen sehr dünnen Spalt ins Magnetfeld eingebracht sein. Dort sind der magnetische Fluss, der Gradient und die resultierende induzierte Spannung höher als in einem breiten Spalt.

Die Ausführung der Erfindung ist nicht auf die oben beschriebenen Beispiele und hervorgehobenen Aspekte der Erfindung beschränkt, sondern ebenso in einer Vielzahl von Abwandlungen möglich, die im Rahmen er folgenden Ansprüche liegen.

## Patentansprüche

1. Aktives medizinisches Implantat (1), insbesondere medizinelektronisches Gerät, mit einer Energieversorgung, die einen mechanischen Schwinger (10, 10a, 10b) aufweist, der durch Bewegungen des Implantat-Trägers (405) und/oder äußere Anregung (410, 411, 521) in Schwingung (401-403, 406) versetzt wird und energetisch derart mit einer Implantatelektronik (41) und/oder einem Energiespeicher (42) verbunden ist, dass ein Teil der mit der Schwingung erzeugten kinetischen Energie in, den Verbraucher und/oder Energiespeicher eingekoppelt wird, **dadurch gekennzeichnet, dass** der oder ein Schwinger teilweise aus elektrischen Komponenten der Implantatelektronik (41) oder des Energiereservoirs (42) besteht.

2. Implantat nach Anspruch 1, wobei der Energiespeicher (42) einen Speicher elektrischer Energie, insbesondere Akku oder Kondensator, aufweist und dem Schwinger (10, 10a, 10b) ein mechanisch-elektrischer Wandler (30, 30a, 30b, 320, 321, 322) zugeordnet ist.

3. Implantat nach Anspruch 1, wobei der Energiespeicher (42) einen Speicher mechanischer Energie, aufweist und diesem ein mechanisch-elektrischer Wandler (30, 30a, 30b, 320, 321, 322) zur Umwandlung gespeicherter mechanischer Energie in elektrische Betriebsenergie des Verbrauchers (41) zugeordnet ist.

4. Implantat nach Anspruch 2 oder 3, wobei der mechanisch-elektrische Wandler (30, 30a, 30b) ein piezoelektrisches Element (320) aufweist.

5. Implantat nach Anspruch 2 oder 3, wobei der mechanisch-elektrische Wandler (30, 30a, 30b) einen elektromagnetischen Wandler, insbesondere eine Spulen-Magnet-Anordnung (321, 322) aufweist.

6. Implantat nach einem der vorangehenden Ansprüche, wobei der Schwinger (10, 10a, 10b) mit elektrischen Wirkkomponenten (41) des Implantats (1) in einem gemeinsamen Gehäuse angeordnet ist.

7. Implantat nach einem der vorangehenden Ansprüche, wobei mehrere Schwinger (10a, 10b) mit unterschiedlichen Schwingungsrichtungen bzw. Bewegungsachsen und/oder unterschiedlicher Eigenfrequenz vorgesehen sind.

8. Implantat nach einem der vorangehenden Ansprüche, wobei der oder ein Schwinger (10, 10a, 10b) in mehr als einer Richtung, insbesondere dreidimensional, schwingungsfähig ausgebildet ist.

9. Implantat nach einem der vorangehenden Ansprüche, wobei der oder ein Schwinger (10, 10a, 10b) eine selbsttätig veränderliche Schwingungsrichtung aufweist.

10. Implantat nach Anspruch 9, wobei der Schwinger (10, 10a, 10b) mit veränderlicher Schwingungsrichtung Mittel zur Einstellung der Schwingungsrichtung in Abhängigkeit von der Körperlage und/oder von Bewegungen (405) des Implantat-Trägers aufweist.

11. Implantat nach einem der vorangehenden Ansprüche, wobei der oder jeder Schwinger (10, 10a, 10b) zur Aufnahme einer äußeren Anregung mit einer auf seine Eigenfrequenz abgestimmten Frequenz ausgebildet ist.

12. Implantat nach Anspruch 11, wobei dem äußerlich anregbaren Schwinger (10, 10a, 10b) ein elektrisch-mechanischer Wandler (30, 30a, 30b), insbesondere ein Piezoelement (320) oder elektromagnetischer Wandler (321, 322), zur Anregung durch ein äußeres elektromagnetisches Wechselfeld zugeordnet ist.

13. Implantat nach Anspruch 1, wobei mehrere bewegliche, elektrische Verbindungen (331) zwischen dem teilweise aus elektrischen Komponenten der Implantatelektronik (41) bestehenden Schwinger (10, 10a, 10b) und den fest installierten Teilen der Implantatelektronik vorhanden sind.

## Claims

1. An active medical implant (1), in particular a medical electronic device, comprising a power supply, which has a mechanical vibrator (10, 10a, 10b), which is induced to vibrate (401-403, 406) by movements of a patient wearing the implant (405) and/or by external excitation (410, 411, 521) and is energetically connected to an implant electronics unit (41) and/or an energy store (42), such that a portion of the kinetic energy generated with the vibration is coupled into the consumer and/or energy store, **characterised in that** the vibrator or a vibrator consists in part of electrical components of the implant electronics unit (41) or of the energy reservoir (42).

2. The implant according to claim 1, wherein the energy store (42) has a store of electrical energy, in particular a battery or capacitor, and the vibrator (10, 10a, 10b) is assigned a mechanical-electrical converter (30, 30a, 30b, 320, 321, 322).

3. The implant according to claim 1, wherein the energy store (42) has a store of mechanical energy, and this is assigned a mechanical-electrical converter (30, 30a, 30b, 320, 321, 322) for converting stored mechanical energy into electrical operating power for the consumer (41).

4. The implant according to claim 2 or 3, wherein the mechanical-electrical converter (30, 30a, 30b) has a piezoelectric element (320).

5. The implant according to claim 2 or 3, wherein the mechanical-electrical converter (30, 30a, 30b) has an electromagnetic converter, in particular a coil-magnet arrangement (321, 322).

6. The implant according to any one of the preceding claims, wherein the vibrator (10, 10a, 10b) is arranged in a shared housing with operative electrical components (41) of the implant (1).

7. The implant according to any one of the preceding claims, wherein a plurality of vibrators (10a, 10b) are provided with different vibration directions or movement axes and/or different natural frequencies.

8. The implant according to any one of the preceding claims, wherein the one or more vibrators (10, 10a, 10b) is/are capable of vibrating in more than one direction, in particular three-dimensionally.

9. The implant according to any one of the preceding claims, wherein the one or more vibrators (10, 10a, 10b) has/have an independently variable direction of vibration.

10. The implant according to claim 9, wherein the vibrator (10, 10a, 10b) with the variable direction of vibration has means for adjusting the direction of vibration as a function of the body position and/or movements (405) of the patient wearing the implant.

11. The implant according to any one of the preceding claims, wherein the, or each vibrator (10, 10a, 10b) is designed to receive external excitation at a frequency tuned to its natural frequency.

12. The implant according to claim 11, wherein externally excitable vibrator (10, 10a, 10b) is assigned an electrical-mechanical converter (30, 30a, 30b), in particular a piezoelement (320) or electromagnetic converter (321, 322), for excitation by an external electromagnetic alternating field.

13. The implant according to claim 1, wherein a plurality of movable, electrical connections (331) are provided between the vibrator (10, 10a, 10b), consisting in part of electrical components of the implant electronics unit (41), and the fixedly installed parts of the implant electronics unit.

## Revendications

1. Implant médical actif (1), notamment appareil médical électronique, avec une alimentation en énergie qui présente un vibreur mécanique (10, 10a, 10b) qui est mis en mode vibratoire (401 à 403, 406) par des mouvements du porteur de l'implant (405) et/ou par une sollicitation extérieure (410, 411, 521) et est reliée du point de vue énergétique avec un système électronique d'implant (41) et/ou un accumulateur d'énergie (42) de telle manière qu'une partie de l'énergie cinétique générée avec la vibration est mise à contribution dans l'utilisateur et/ou dans l'accumulateur d'énergie, **caractérisé en ce que** le ou un vibreur est constitué partiellement de composants électriques du système électronique d'implant (41) ou du réservoir d'énergie (42).

2. Implant selon la revendication 1, dans lequel l'accumulateur d'énergie (42) présente un système de stockage d'énergie électrique, notamment un accumulateur ou un condensateur et qu'un convertisseur mécanique-électrique (30, 30a, 30b, 320, 321, 322) est associé au vibreur (10, 10a, 10b).

3. Implant selon la revendication 1, dans lequel l'accumulateur d'énergie (42) présente un accumulateur d'énergie mécanique et qu'un convertisseur mécanique-électrique (30, 30a, 30b, 320, 321, 322) est associé à celui-ci pour la conversion d'énergie mécanique stockée en énergie électrique opérationnelle de l'utilisateur (41).

4. Implant selon la revendication 2, ou 3, dans lequel le convertisseur mécanique-électrique (30, 30a, 30b) présente un élément piézoélectrique (320).

5. Implant selon la revendication 2, ou 3, dans lequel le convertisseur mécanique-électrique (30, 30a, 30b) présente un convertisseur électromagnétique, notamment un ensemble bobine-aimant (321, 322).

6. Implant selon l'une des revendications précédentes, dans lequel le vibreur (10, 10a, 10b) est disposé dans un boîtier commun avec des composants actifs électriques (41) de l'implant (1).

7. Implant selon l'une des revendications précédentes, dans lequel plusieurs vibreurs (10a, 10b) avec des directions de vibrations, respectivement des axes de déplacement, différents et/ou fréquences propres différentes, sont prévus.

8. Implant selon l'une des revendications précédentes, dans lequel le ou un vibreur (10, 10a, 10b) est conçu pour être capable de vibrer dans plus d'une direction, notamment de manière tridimensionnelle.

9. Implant selon l'une des revendications précédentes, dans lequel le ou un vibreur (10, 10a, 10b) présente une direction de vibration variable automatiquement.

10. Implant selon la revendication 9, dans lequel le vibreur (10, 10a, 10b) présente des moyens avec une direction de vibration variable pour le réglage de la direction de vibration en fonction de la position du corps et/ou des mouvements (405) du porteur d'implant.

11. Implant selon l'une des revendications précédentes, dans lequel le ou chaque vibreur (10, 10a, 10b) est conçu pour la réception d'une sollicitation extérieure avec une fréquence adaptée à sa fréquence propre.

12. Implant selon la revendication 11, dans lequel un convertisseur électrique-mécanique (30, 30a, 30b), notamment un élément piézoélectrique (320), ou un convertisseur électromagnétique (321, 322) est associé au vibreur (10, 10a, 10b) pouvant être sollicité de l'extérieur pour l'activation par un champ électromagnétique alternatif externe.

13. Implant selon la revendication 1, dans lequel plusieurs connexions électriques (331) mobiles sont présentes entre le vibreur (10, 10a, 10b) constitué partiellement de composants électriques du système électronique (41) et les parties installées fixes du système électronique d'implant.
